# EUROPEAN PATENT APPLICATION

(11) **EP 2 719 315 A1**
(43) Date of publication of application: **16.04.2014**
(21) Application number: 12796747.9
(22) Date of filing: 05.06.2012
(51) Int. Cl.: A61B 1/00, A61B 1/04, G02B 7/00, G02B 23/26

(54) **ELECTRONIC ENDOSCOPE DEVICE AND MANUFACTURING METHOD THEREFOR**

(30) Priority: 07.06.2011 JP 2011127482; 17.05.2012 JP 2012113425
(71) Applicant: FUJIFILM Corporation, Tokyo 106-8620 (JP)
(72) Inventor: MIZUYOSHI Akira, Ashigarakami-gun Kanagawa 258-8538 (JP)
(74) Representative: Klunker . Schmitt-Nilson . Hirsch
(86) International application number: PCT/JP2012/064419
(87) International publication number: WO 2012/169476

(57) **Abstract**

An electronic endoscope device with enhanced heat resistance and durability is provided. In the electronic endoscope device, an optical member, and a frame body made of a material having a thermal expansion coefficient different from the optical member and holding the optical member are arranged at an insertion section to be inserted into a subject. The optical member and the frame body are connected to each other via adhesive layers of a three-layer structure having mutually different thermal expansion coefficients.

## Description

### BACKGROUND OF THE INVENTION

### 1. Field of the Invention

The present invention relates to an electronic endoscope device and a manufacturing method thereof.

### 2. Description of the Related Art

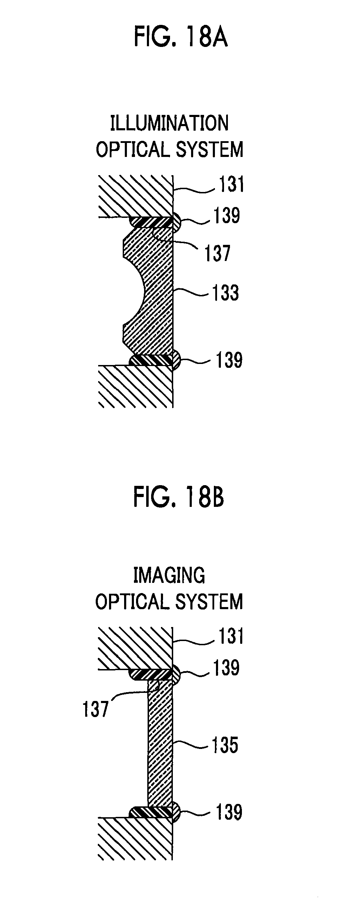

Endoscopes have an insertion section to be inserted into a body cavity, and a tip of the insertion section is provided with an illumination optical system that emits illumination light on a subject, and an imaging optical system that forms a subject image. For fixing of an optical member, such as a lens 133 of the illumination optical system exposed to the outside at a tip 131 of the insertion section as shown in Fig. 18A or a cover glass 135 of the imaging optical system as shown in Fig. 18B, a method of filling an adhesive material 137 into a gap between the optical member and a holding portion that houses the optical member and fixing the optical member and the holding portion as a sealing structure is adopted.

In the sealing structure of the illumination optical system or the imaging optical system as described above, an epoxy-based adhesive material or a silicon-based adhesive material is used for the gap between a side surface of the optical member and the holding portion. Additionally, a bank portion 139 is provided on an outer peripheral edge of the optical member on the outer side thereof for prevention of flares, and a blackened epoxy-based adhesive material is used for the bank portion 139.

However, the epoxy-based adhesive material or the silicon-based adhesive material has relatively high moisture permeability, and even as the sealing structure, moisture will permeate into a layer of the adhesive material 137 when the endoscope is cleaned. For this reason, a cleaning material or the like may soak into the endoscope and internal contamination may be caused. Additionally, the epoxy-based adhesive material may not necessarily have sufficient strength against rubbing during wiping using a cleaning brush or a gauze, and the epoxy bank portion 139 may be shaved off and shielding of unnecessary light may become insufficient, or moisture may enter from the shaved-off region.

In addition, typically when an optical member made of glass or the like and a metallic holding portion are connected together, the thermal expansion coefficient of the optical member is smaller in comparison to that of metal, and the difference between the thermal expansion coefficients is large. Therefore, reducing the influence caused by the difference between the thermal expansion coefficients of these respective materials is performed. In this case, restrictions on material selection of the optical member, the holding portion, and adhesive material connecting both increase. A configuration in which a metallic holding portion having a smaller thermal expansion coefficient and an optical member made of glass are heated and sealed with low-melting-point glass is described in JP2005-227728A. Even in case, however, it is difficult to select and connect a high chemical-resistant optical member, and there is also a disadvantage in that the machinability of the metallic holding portion deteriorates.

### SUMMARY OF THE INVENTION

Whenever medical endoscope devices are inserted into a body cavity and used, cleaning, disinfection, and sterilization processing are performed in order to remove adhered contaminants. In recent years, implementation of autoclave processing that performs cleaning with high-temperature and high-pressure steam exceeding 100°C has been desired in order to more reliably prevent occurrence of infection. In endoscope devices exposed under such a severe condition, particularly a bonded portion is apt to receive damage, such as thermal stress, and therefore, further heat resistance or durability is required.

In the medical endoscope devices, electronic components, such as an imaging element, are mounted on a tip portion of an endoscope insertion section. For this reason, if moisture enters up to the electronic component during the autoclave processing, the endoscope insertion section may fail and require replacement. In the medical endoscope devices, it is necessary to avoid a situation where the endoscope insertion section is frequently replaced, and even being exposed under severe environments, such as the autoclave processing, development of endoscope devices that do not fail easily is required.

An object of the invention is to provide an electronic endoscope device and a manufacturing method thereof that can enhance heat resistance and durability under severe conditions, such as autoclave processing.

An electronic endoscope device of the invention includes: an optical member; and a frame body made of a material having a thermal expansion coefficient different from the optical member and holding the optical member. The optical member and the frame body are arranged at a tip portion of an insertion section inserted into a subject. An electronic component including an imaging element is built in the tip portion. The optical member and the frame body are connected to each other via three or more adhesive layers having mutually different thermal expansion coefficients.

In a manufacturing method of an electronic endoscope device of the invention, a second adhesive material layer forming step is a step of providing a stepped portion, which is enlarged in diameter by stepping, at one end portion of the inner peripheral surface of the frame body, and forming the second adhesive material layer on the diameter-enlarged inner peripheral surface of the stepped portion; a third adhesive material layer forming step is a step of forming a third adhesive material layer outside a first adhesive material layer, and in an inserting step, the outer peripheral surface of the optical member is pressed against the second adhesive material layer, which is formed on the diameter-enlarged inner peripheral surface of the stepped portion, from an insertion direction of the optical member whereby the second adhesive material layer is made to extend to an annular side surface of the frame body formed by the stepping; and a second adhesive material layer is formed at a gap between the outer peripheral surface of the optical member and the diameter-enlarged inner peripheral surface of the stepped portion that faces the outer peripheral surface and a gap between the annular side surface and an outer peripheral edge of the optical member that faces the annular side surface over the whole circumferences thereof, respectively.

According to the electronic endoscope device of the invention, heat resistance and durability under severe conditions, such as autoclave processing, can be enhanced.

### BRIEF DESCRIPTION OF THE DRAWINGS

Fig. 1 is an overall view of an endoscope illustrating an embodiment of the invention.
Fig. 2 is a perspective view of an endoscope tip portion.
Fig. 3 is a schematic cross-sectional view showing cross-section A-A of Fig. 2.
Fig. 4A is a schematic configuration view of an illumination optical system that guides emission light from a white light source to the endoscope tip portion with an optical fiber bundle, and Fig. 4B is a schematic configuration view of the illumination optical system that guides laser light to a fluorescent body of the endoscope tip portion with a single-wire optical fiber.
Fig. 5 is a schematic cross-sectional view showing that an optical member is fixed to an opening hole of a tip rigid portion via a plurality of adhesive layers.
Figs. 6A, 6B, and 6C are process drawings showing a procedure for fixing the optical member to the opening hole of the tip rigid portion via the plurality of adhesive layers.
Fig. 7 is an explanatory view showing the distribution of thermal stress generated due to temperature changes in a joining portion between the tip rigid portion and the optical member.
Fig. 8 is a schematic cross-sectional view showing that an optical member made of a sapphire plate is fixed to the opening hole of the tip rigid portion via the plurality of adhesive layers.
Fig. 9 is an explanatory view schematically showing an aspect where a first adhesive material preform is made.
Fig. 10A is an explanatory view showing the process of inserting the preform into an outer peripheral surface of the optical member, and Fig. 10B is an explanatory view showing the process of inserting the preform into the opening hole of the tip rigid portion.
Fig. 11 is a schematic cross-sectional view showing that the optical member is fixed to the opening hole of the tip rigid portion via three adhesive layers.
Fig. 12 is a view showing a state where a crack is generated in a sealing structure shown in Fig. 11.
Fig. 13 is a schematic cross-sectional view showing that the optical member is fixed to a stepped portion of the tip rigid portion via the plurality of adhesive layers.
Fig. 14 is a front view of the optical member.
Figs. 15A, 15B, 15C, and 15D are process drawings of a case where the optical member is fixed to the tip rigid portion, using the sapphire plate as the optical member.
Fig. 16 is an enlarged explanatory view showing an R portion of Fig. 12 in an enlarged manner.
Fig. 17 is a view showing experiment examples of Configuration Examples 1 to 6 of the sealing structure between the optical member and the tip rigid portion.
Fig. 18A is a configuration view showing a joining structure of lenses of an illumination optical system of the related art, and Fig. 18B is a configuration view showing a joining structure of cover glasses of an imaging optical system of the related art.

### DESCRIPTION OF THE PREFERRED EMBODIMENTS

An embodiment of the invention will be described below in detail referring to the drawings.

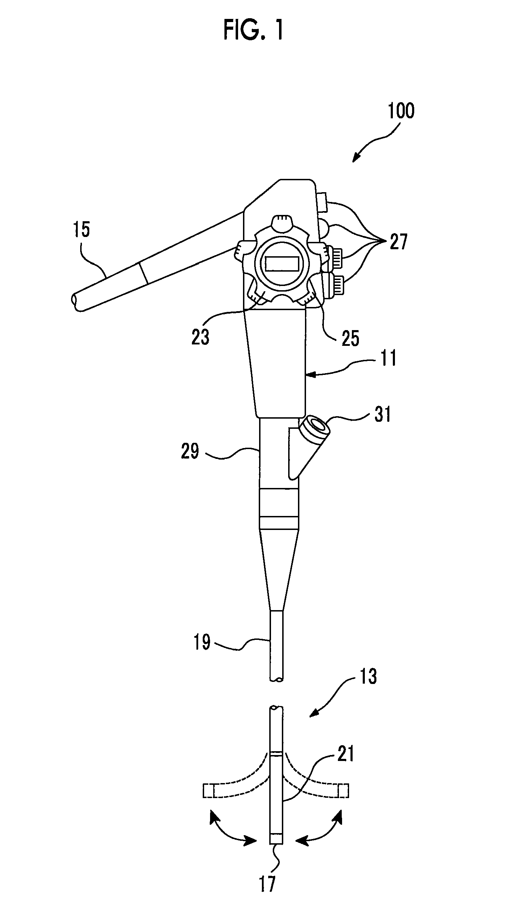

Fig. 1 is a view for describing the embodiment of the invention.

An endoscope 100, which is a medical device, includes a body operating section 11, and an endoscope insertion section 13 that is provided continuously with the body operating section 11 and is inserted into a body cavity. A universal cord 15 is connected to the body operating section 11, and a light guide connector (not shown) is provided at the tip of the universal cord 15. The light guide connector is detachably coupled to a light source device (not shown), and illumination light is sent to an illumination optical system of a tip portion 17 of the endoscope insertion section 13. Additionally, an electric connector is connected to the light guide connector, and the electric connector is detachably coupled to a processor that performs image signal processing or the like.

The endoscope insertion section 13 is constituted by a flexible portion 19, a bending portion 21, and the tip portion (hereinafter also henceforth referred to as an endoscope tip portion) 17 sequentially from the body operating section 11 side. The bending portion 21 can be remotely operated for bending by rotating angle knobs 23 and 25 of the body operating section 11, and thereby can direct the tip portion 17 to a desired direction.

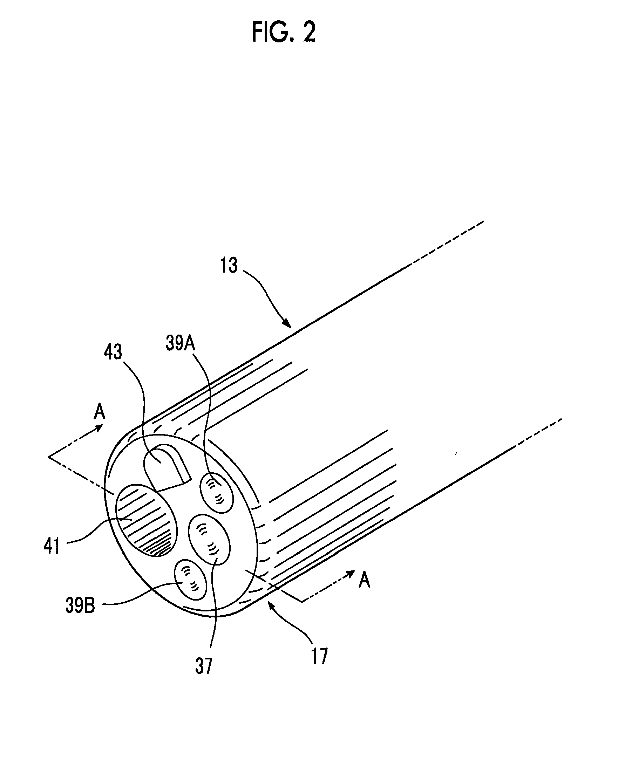

Various buttons 27, such as an air/water feed button, a suction button, and a shutter button, in addition to the aforementioned angle knobs 23 and 25, are provided side by side at the body operating section 11. Additionally, a forceps insertion portion 31 into which, treatment tools, such as forceps, are inserted is provided at a continuously-provided portion 29 that extends from the body operating section 11 to the endoscope insertion section 13 side. The tip of the treatment tool inserted from the forceps insertion portion 31 is led out from a forceps port 41 (refer to Fig. 2) formed in the endoscope tip portion 17.

A schematic appearance view of the endoscope tip portion is shown in Fig. 2. An observation window 37 of an imaging optical system is arranged at a tip surface of the endoscope tip portion 17, and irradiation windows 39A and 39B of an illumination optical system are arranged on both sides of the observation window 37. Additionally, an air/water feed nozzle 43 that feeds air or water toward the forceps port 41 or the observation window 37 is also arranged at the tip surface.

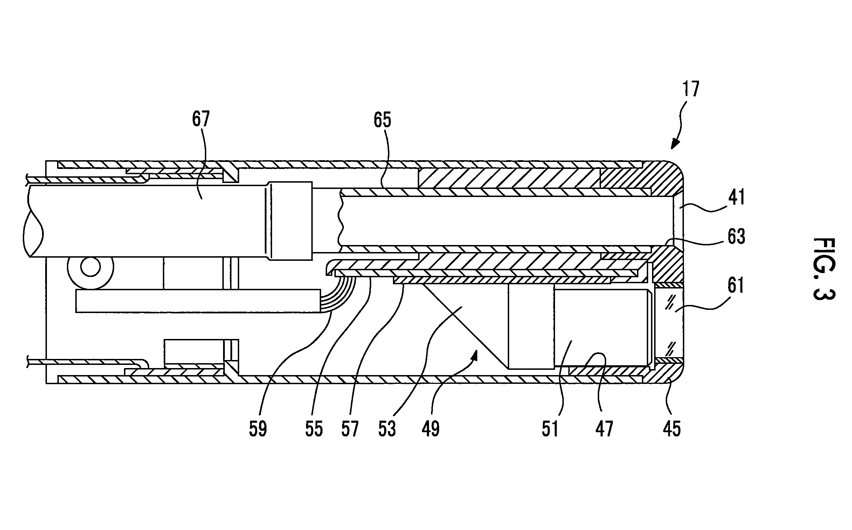

A schematic cross-sectional view showing cross-section A-A of Fig. 2 is shown in Fig. 3.

The endoscope tip portion 17 has a tip rigid portion 45 made of a metallic material, such as a stainless-steel material, or ceramic. A lens barrel 51 of an imaging unit 49 is inserted into an opening hole 47 bored in the tip rigid portion 45, and the imaging optical system is arranged at the opening hole. The imaging unit 49 curves the optical axis of the lens barrel 51 at a right angle with a prism 53 to cause an image to be formed on an imaging element 57 mounted on a substrate 55. An imaging signal from the imaging element 57 is transmitted from the substrate 55 through a signal line 59 to a control device (not shown). A cover glass 61 is provided ahead of an optical path of the lens barrel 51, and a sealing structure is formed between the cover glass and the tip rigid portions 45.

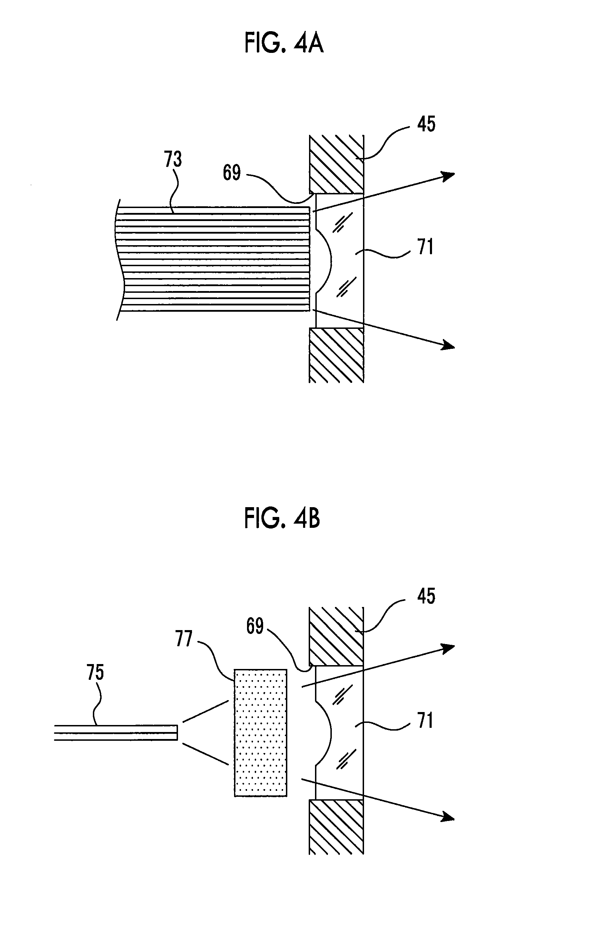

Additionally, also in the illumination optical system, similarly, as shown in Figs. 4A and 4B, a lens 71 that emits illumination light from the endoscope tip portion 17 is inserted into an opening hole 69 bored in the tip rigid portion 45. The illumination optical system has a configuration in which the above lens 71 is arranged at a light emission end of an optical fiber bundle 73, in a configuration in which the emission light from a white light source, such as a xenon lamp or a halogen lamp is guided to the endoscope tip portion 17 with the optical fiber bundle 73 (Fig. 4A).

Additionally, the illumination optical system has a configuration in which the above lens 71 is arranged on the side opposite to a laser light incidence side of a fluorescent body 77 in a case where laser light is guided to the endoscope tip portion 17 with a single-wire optical fiber 75, the fluorescent body 77 is arranged at a light emission end of the optical fiber 75, and the illumination light is generated by the fluorescence from the fluorescent body 77 excited by the laser light and the laser light passes through the fluorescent body 77 (Fig. 4B). In any case, a sealing structure is formed between the lens 71 and the tip rigid portion 45.

As shown in Fig. 3, a metallic forceps pipe 65 is fixedly provided at an opening hole 63 bored in the tip rigid portion 45, and a forceps tube 67 is connected to the end portion of the forceps pipe 65 opposite to the opening hole 63. The forceps pipe 65 and the forceps tube 67 form a forceps channel that communicates from the forceps port 41 of the endoscope tip portion 17 to the forceps insertion portion 31 on the body operating section 11 side.

Next, a sealing structure when an optical member, such as the cover glass 61 or the lens 71, is fixed to the opening hole 47 or 69 of the tip rigid portion 45 will be described in detail.

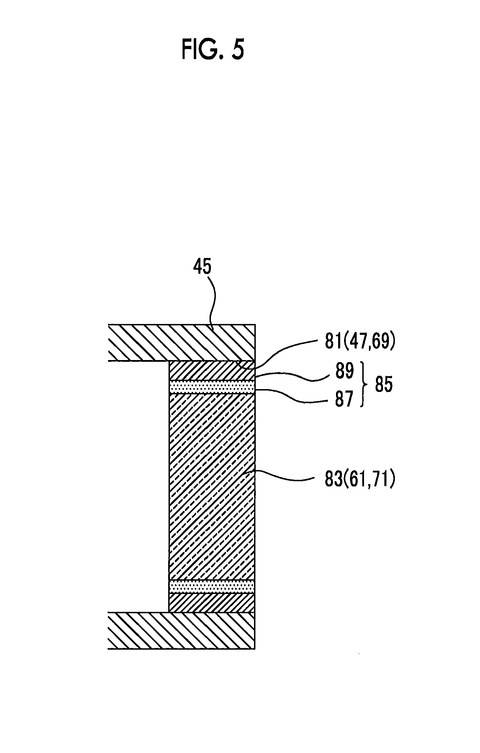

Fig. 5 is a schematic cross-sectional view when an optical member 83 (61, 71) is fixed to an opening hole 81 (47, 69) of the tip rigid portion 45 via a plurality of adhesive layers. In addition, in the following description, the shape of the tip rigid portion 45 will be simplified and described as a simple cylindrical shape.

An optical member, such as a lens or a cover plate having translucency, is arranged at the observation window or illumination window of the endoscope tip portion so as to be exposed to the surface of the endoscope tip portion.

The optical member 83 is held by the opening hole 81 of the tip rigid portion 45 that is a frame body, and the optical member 83 and the opening hole 81 are connected to each other via the plurality of adhesive layers 85 having mutually different thermal expansion coefficients.

The plurality of adhesive layers 85 are laminated with a gap between the optical member 83 and the opening hole 81 in a thickness direction of the gap. The adhesive layers 85 includes a first adhesive material layer 87 on the optical member side, and a second adhesive material layer 89 on the opening hole 81 side. The first adhesive material layer 87 is formed on an outer peripheral surface of the optical member 83 formed in a plate shape, and the second adhesive material layer 89 is formed on an inner peripheral surface of the opening hole 81 that faces the outer peripheral surface.

For example, the first adhesive material layer 87 is made of a first adhesive material having a thermal expansion coefficient that is smaller than that of the tip rigid portion 45 and larger than that of the optical member 83. For example, the second adhesive material layer 89 is made of a second adhesive material having a thermal expansion coefficient that is larger than that of the first adhesive material and smaller than that of the tip rigid portion 45.

The first adhesive material and the second adhesive material contain low-melting-point glass frit made of bismuth-based glass as a filler (spherical melting silica). Here, the bismuth-based glass refers to glass whose main component is bismuth-based glass. Specifically, the first adhesive material is glass paste containing Bi₂O₃-ZnO-B₂O₃-based glass frit, and the thermal expansion coefficient α₁ thereof is 8.5 x 10⁻⁶ [°C⁻¹] (100°C to 300°C). In addition, the term "thermal expansion coefficient" is a value after being sintered and solidified.

The second adhesive material is glass paste containing Bi₂O₃-ZnO-B₂O₃-based glass frit, and the thermal expansion coefficient α₂ thereof is 11.5 x 10⁻⁶ [°C⁻¹] (100°C to 300°C). The first adhesive material is obtained by mixing, composing, and preparing additives, such as zinc oxide, alumina, a small amount of boron oxide, and tin oxide, in the second adhesive material.

The composition of the above low-melting-point glass can be represented by mol% as follows:
BiO₂: 70 to 80%
SiO₂: 1 to 10%
ZnO: 1 to 10%
Al₂O₃: 1 to 10%
B₂O₃: 1 to 10%

When the tip rigid portion 45 is made of stainless steel (SUS304), the thermal expansion coefficient α_{f} is 17.3 x 10⁻⁶ [°C⁻¹].

The optical member 83 is made of glass (for example, K-LASFN17 (trade name, made by Sumita Optical Glass Inc.)), and the thermal expansion coefficient α_{OP} is 7.9 x 10⁻⁶ [°C⁻¹]

(100°C to 300°C). Hence, the relationship between the thermal expansion coefficients of the respective members becomes α_{OP} < α₁ < α₂ < α_{f}. In addition, the magnitude relationship of the thermal expansion coefficients is not limited to the above-described magnitude relationship, and may be, for example, a relationship of α_{OP} < α₁ < α₂ > α_{f}, or α_{OP} < α₁ > α₂ < α_{f}. That is, the plurality of adhesive materials may have mutually different thermal expansion coefficients.

Next, the process of fixing the optical member 83 to the opening hole 81 of the tip rigid portion 45 will be described.

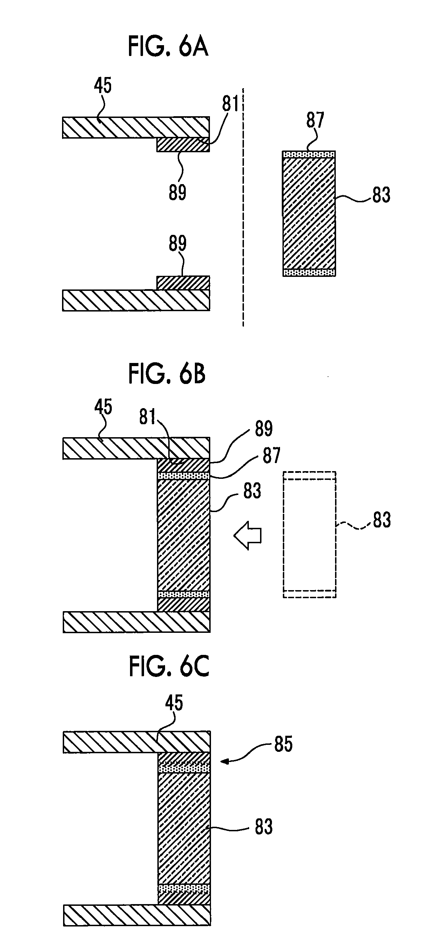

A procedure for fixing the optical member 83 to the opening hole 81 of the tip rigid portion 45 via the plurality of adhesive layers is shown in Figs. 6A, 6B, and 6C.

Fig. 6A shows an aspect where the paste-like second adhesive material is heated to form the second adhesive material layer 89 after being coated on the inner peripheral surface of the opening hole 81 of the tip rigid portion 45, and an aspect where the paste-like first adhesive material is heated to form the first adhesive material layer 87 after being coated on the outer peripheral surface of the optical member 83.

By heating the first adhesive material and second adhesive material at 200 to 300°C lower than a glass softening point (about 400°C) and holding the first and second adhesive materials for 30 minutes or more, an organic binder contained in the glass paste or a diluted solution used, if necessary, during coating is burned off to form the respective layers 87 and 89. Here, heating and holding may be further made at 450°C or higher so as to bake the glass in the respective layers 87 and 89.

Next, as shown in Fig. 6B, the optical member 83 is inserted into and combined with the opening hole 81 of the tip rigid portion 45. Since the first adhesive material and second adhesive material are solidified, the respective adhesive material layers are joined together without becoming out of order during insertion. Then, by performing baking at 450 to 500°C higher than the glass softening point for 20 minutes or more, as shown in Fig. 6C, the optical member 83 and the tip rigid portion 45 are sintered via the adhesive layers 85.

The interface between the first adhesive material layer 87 and the second adhesive material layer 89 after the sintering forms a boundary region with a predetermined width due to inter-penetration in practice, and has a distribution in which the layers change continuously within the boundary region.

As described above, by sintering the optical member 83 at the tip rigid portion 45, using low-melting-point glass having different thermal expansion coefficients for every layer, a sealing structure having no permeability of moisture and having sufficient heat resistance and durability is obtained.

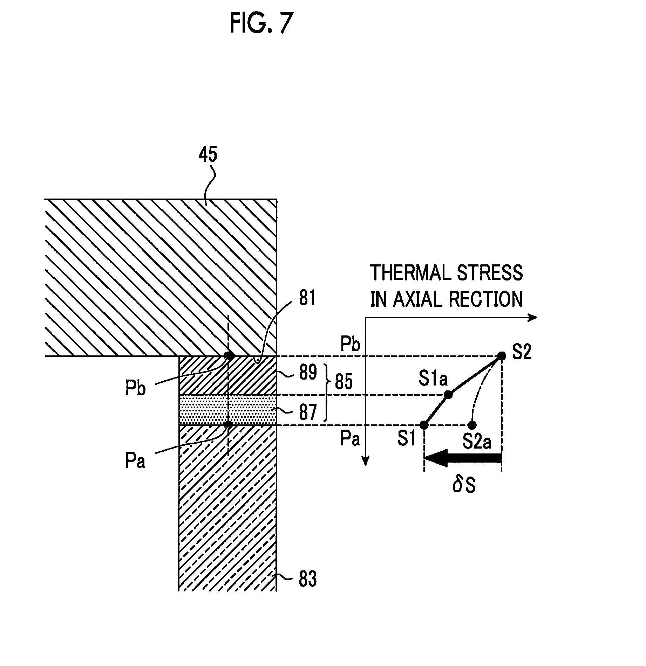

Fig. 7 is an explanatory view showing the distribution of thermal stress generated due to temperature changes in a joining portion between the tip rigid portion 45 and the optical member 83. The thermal stress of the optical member 83 in the radial direction (a direction of a straight line Pa-Pb) on a laminating line of the optical member 83, the first adhesive material layer 87, the second adhesive material layer 89, and the tip rigid portion 45 which is shown by the straight line Pa-Pb, can be relieved by the first adhesive material layer 87 and the second adhesive material layer 89.

That is, since a large heat distortion is caused by thermal expansion on the tip rigid portion 45 side with a large thermal expansion coefficient when a predetermined amount of temperature rise has occurred, a large thermal stress shown by S2 is generated. On the other hand, although the amount of thermal expansion is small on the optical member 83 side with a small thermal expansion coefficient, the optical member 83 receives a tensile stress in a direction going from Pa to Pb due to the expansion of the tip rigid portion 45. However, the amounts of thermal expansion of the first adhesive material layer 87 and the second adhesive material layer 89 gradually become a middle amount of thermal expansion, whereby the tensile stress to be generated is gradually reduced compared to a case where the tip rigid portion 45 is directly connected to the optical member 83. This can reliably prevent breaking in the junction interface, rupture of the respective layers, or the like.

In the present configuration, as the adhesive layers include a plurality of layers, the thermal stress to be generated can be relieved compared to a case where the tip rigid portion 45 and the optical member 83 are joined together by a single layer. The gap between the tip rigid portions 45 and the optical member 83 that is required for medical electronic endoscopes is about 0.01 to 0.2 mm. When materials having largely different thermal expansion coefficients are joined together within such narrow thickness, the relieving effect of substantial thermal stress is small even if a material having a middle thermal expansion coefficient between the thermal expansion coefficients of both the materials is interposed as a single layer between both the materials.

By attempting to interpose the plurality of adhesive layers in a narrow gap from the tip rigid portion 45 side to the optical member 83 side as in the present configuration, a thermal stress reduction effect that is not obtained from a single adhesive layer is exhibited. The above effect becomes more marked as the dimension of the gap is narrower, and becomes marked at 0.01 to 0.1 mm, particularly, 0.01 to 0.05 mm.

As shown in Fig. 7, a thermal stress S2 on the tip rigid portion 45 side is reduced to the thermal stress S1a on the side opposite to the tip rigid portion 45 side by the second adhesive material layer 89, and is further reduced from the thermal stress S1a to a thermal stress S1 by the first adhesive material layer 87. On the other hand, when the adhesive layer is a single-layer structure, the thermal stress value becomes a gentle curve, and remains at the amount of stress relief to a thermal stress S2a that is smaller than the amount δS of stress relief from the thermal stress S2 to the thermal stress S1, as shown by a one-dot chain line in the drawing.

First, one layer out of the adhesive layers that face each other hardens earlier than the other layer at the interface between the adhesive layers of different kinds of materials in the present configuration. Next, the glass of the other layer hardens on the one layer that is hardened, and both the layers are bonded together. When the other layer hardens, the stress is released in the direction of the interface so as to produce a stress relief.

As described above, by connecting the optical member 83 and the tip rigid portion 45 together via the plurality of adhesive layers 85 of low-melting-point glass with mutually different thermal expansion coefficients, the optical member 83, and the tip rigid portion 45 that supports the optical member 83 can be sealed with enhanced heat resistance and durability. Additionally, since complicated processes are not accompanied, workability is not impaired.

### <Modification Example of Optical Member>

The above optical member 83 can also be formed from single crystal sapphire in addition to glass. By forming the optical member 83 from high-strength single crystal sapphire, the thickness of the optical member 83 can be reduced to, for example, about 0.15 mm to 0.3 mm (1 to 2 mm in diameter).

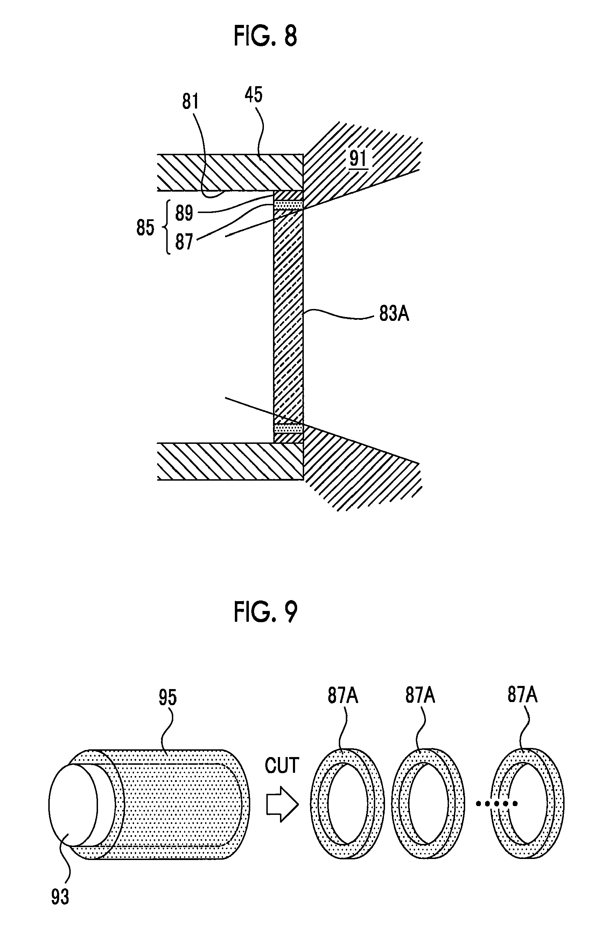

Fig. 8 is a schematic cross-sectional view showing that an optical member made of a sapphire plate 83A is fixed to the opening hole 81 of the tip rigid portion 45 via the plurality of adhesive layers 85. Since the sapphire plate 83A can be thinned, the thickness of the plurality of adhesive layers 85, such as the first adhesive material layer 87 and the second adhesive material layer 89, can be reduced. The sapphire plate 83A is an illumination window, has a large light emission area, and is apt to produce vignetting 91 at the tip of the illumination window when illumination light with a large spread angle is emitted therethrough. However, since the adhesive layer 85 is formed thinly, the region of the adhesive layers 85 that produces the vignetting 91 is limited to a small range, and generation of heat from the region is suppressed to the minimum.

In addition, a black pigment may be mixed in the first adhesive material layer 87 in order to prevent stray light. The black pigment includes an isotropic graphite material. Since the thermal expansion coefficient of the isotropic graphite material is 4.4 to 7.1 x 10⁻⁶°C⁻¹ and is approximate to the thermal expansion coefficient of the optical member 83, the isotropic graphite material can be suitably used.

### <Modification Example of Tip Rigid Portion>

Although the material of the aforementioned tip rigid portion 45 has been described as stainless steel (SUS304), a material having a smaller thermal expansion coefficient can be used. As the material of the tip rigid portion 45, for example, Kovar (trade name) (components: Ni 29 wt%, Co 17 wt%, and Fe Bal.,; thermal expansion coefficient: 4.8 x 10⁻⁶(30°C to 400°C)°C⁻¹), 42 Alloy (components: Ni 40.5 wt% and Fe Bal., ; thermal expansion coefficient: 5.0 x 10⁻⁶(30°C to 300°C)°C⁻¹) and NSL (made by the Toshiba Materials Co. Ltd.) (components: Ni 42 wt%, special component, and Fe Bal.,; thermal expansion coefficient: 5.0 x 10⁻⁶(30 to 300°C)°C⁻¹) can be used.

By using an Fe-based material containing at least one of Ni and Co as the material of the tip rigid portion 45 as described above, the thermal expansion coefficient becomes small and a difference in thermal expansion coefficient from the optical member can be made small.

### <Modification Example of First Adhesive Material Layer and Second Adhesive Material Layer>

Next, a method of forming the adhesive layers using adhesive material preforms made in advance will be described. In the above example, the adhesive layers are formed, respectively, by coating the paste-like first adhesive material on the outer peripheral surface of the optical member 83 and coating the second adhesive material on the inner peripheral surface of the opening hole 47 of the tip rigid portion 45. Here, the adhesive layers are formed using annular preforms obtained by solidifying the first adhesive material and the second adhesive material in advance.

Fig. 9 is an explanatory view schematically showing an aspect where the first adhesive material preform is made. First, the paste-like first adhesive material is coated on a side surface of a columnar rod body 93, and is heated to 200 to 300°C lower than the glass softening point. This bums off the organic binder or diluted solution contained in the glass paste, and forms a first adhesive material layer 95 on the side surface of the rod body 93. Thereafter, in a state where the rod body 93 is supported, slicing is performed perpendicularly to the axis of the rod body 93 by a cutter (not shown) so as to cut the layer 95 into a plurality of annular individual pieces. Both side surfaces of the individual pieces are ground to form a preform 87A.

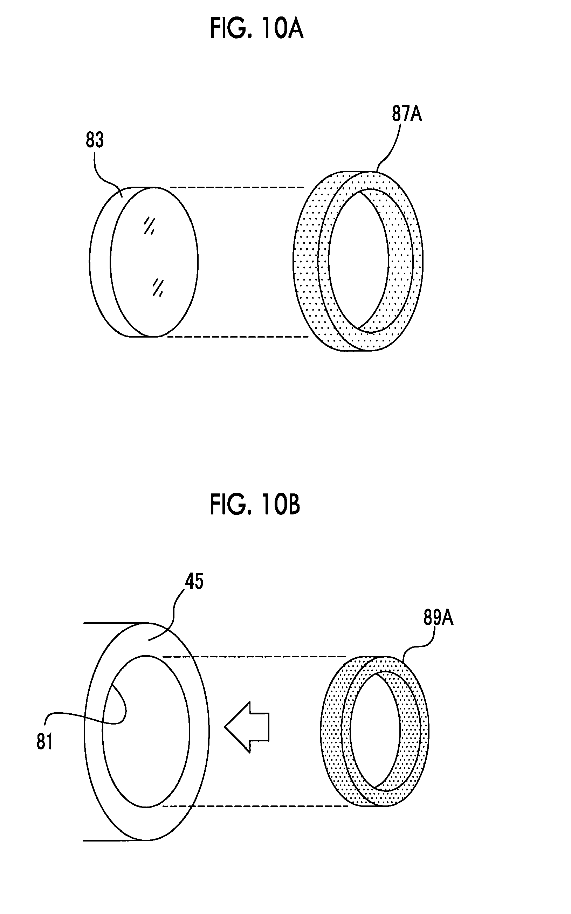

If the preform obtained by solidifying the first adhesive material is prepared in advance as described above, as shown in Fig. 10A, the first adhesive material layer can be simply and rapidly formed by inserting and assembling the preform 87A into the outer peripheral surface of the optical member 83.

By making the second adhesive material preform by the same process and inserting and assembling the already made preform 89A into the opening hole 81 of the tip rigid portion 45 as shown in Fig. 10B, the second adhesive material layer can be formed simply and rapidly.

The method of forming the respective adhesive layers using the preforms is particularly useful for a case where the optical member 83 is a thin sapphire plate and this sapphire plate is fixed to the tip rigid portion. A process example of fixing the sapphire plate is shown below.

(1) The first adhesive material preform is attached to an outer peripheral surface of the sapphire plate that is the optical member 83, and is temporarily fixed by heating. (2) The paste-like second adhesive material is coated on the opening hole of the tip rigid portion. (3) The sapphire plate provided with the first adhesive material preform is fitted into the opening hole of the tip rigid portion. (4) Baking is performed at a temperature equal to or higher than the glass softening point.

According to the above process, the thin sapphire plate that is difficult to handle can be simply and reliably fixed without causing warpage or coming-off.

### <Second Configuration Example>

Next, a configuration example in which a three-layer structure is adopted instead of the adhesive layers of the two-layer structure will be described.

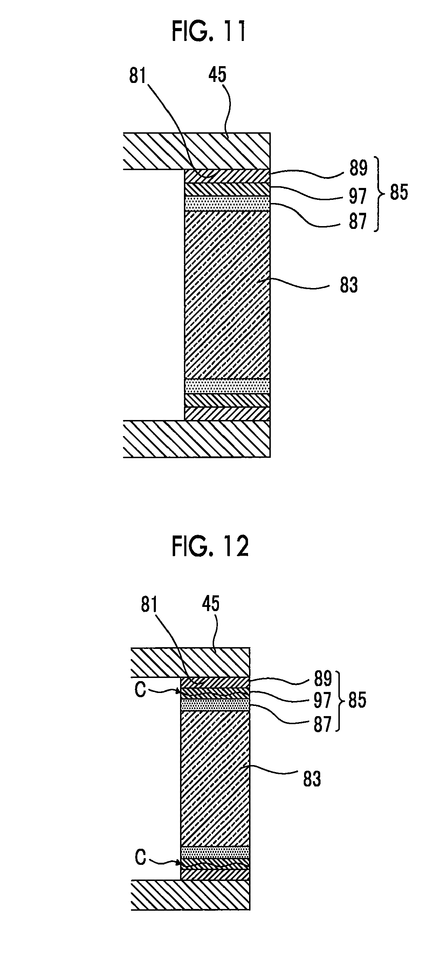

Fig. 11 is a schematic cross-sectional view showing that the optical member 83 is fixed to the opening hole 81 of the tip rigid portion 45 via the three adhesive layers. A third adhesive material layer 97 having a middle thermal expansion coefficient between the thermal expansion coefficients of the first adhesive material and the second adhesive material, for example, is formed between the aforementioend first adhesive material layer 87 and the aforementioned second adhesive material layer 89. The thermal expansion coefficient of the third adhesive material is not limited to a middle value between the thermal expansion coefficient of the first adhesive material and the thermal expansion coefficient of the second adhesive material, and may be, for example, a value larger than the thermal expansion coefficients of the first adhesive material and second adhesive material. That is, the first adhesive material, the second adhesive material, and the third adhesive material may have mutually different thermal expansion coefficients.

The third adhesive material contains as a filler the low-melting-point glass frit made of bismuth-based glass, similar to the aforementioned first and second adhesive materials. Here, the bismuth-based glass refers to glass whose main component is bismuth-based glass. Specifically, the third adhesive material is glass paste containing Bi₂O₃-ZnO-B₂O₃-based glass frit, and the thermal expansion coefficient α₃ thereof is 8.8 x 10⁻⁶ [°C⁻¹] (100°C to 300°C). Additionally, as another example, glass paste (thermal expansion coefficient 12.3 x 10⁻⁶ [°C⁻¹]) containing P₂O₅-ZnO-R₂O-based glass frit may be used as the third adhesive material.

The above three-layer structure can be formed as follows. First, the third adhesive material layer 97 is formed outside the first adhesive material layer 87, and then, is inserted into and sintered in the opening hole 81 formed with the second adhesive material layer 89 of the tip rigid portion 45. Alternatively, the third adhesive material layer 97 is formed inside the second adhesive material layer 89, and then, the optical member 83 formed with the first adhesive material layer 87 is inserted into and sintered in the opening hole 81. Alternatively, the third adhesive material layers 97 are formed outside the first adhesive material layer 87 and inside the second adhesive material layer 89, respectively, and the optical member 83 is inserted into and sintered in the opening hole 81. Since the sintering processing is the same as that of the above-mentioned sintering processing, description thereof will be omitted here.

Fig. 12 is a cross-sectional schematic view of the tip portion showing a result obtained by performing autoclave processing on high-temperature and high-pressure (for example, 134°C and pressure of 2.2 bars) conditions to an endoscope insertion section with the sealing structure including the adhesive layers of the three-layer structure shown in Fig. 11 until a crack is generated in the adhesive layer.

If the autoclave processing is repeatedly performed as shown in Fig. 12, a crack is not generated at an interface between the first adhesive material layer 87 and the third adhesive material layer 97 and an interface between the second adhesive material layer 89 and the third adhesive material layer 97, but a crack C is generated in the third adhesive material layer 97.

This result means that stresses applied to the adhesive layers are concentrated on the third adhesive material layer 97, and stresses applied to an interface between the first adhesive material layer 87 and the optical member 83, the interface between the first adhesive material layer 87 and the third adhesive material layer 97, the interface between the second adhesive material layer 89 and the third adhesive material layer 97, and an interface between the second adhesive material layer 89 and the tip rigid portion 45 are reduced.

In addition, the crack C as shown in Fig. 12 is generated when the autoclave processing is carried out about 500 to 2000 times, and a crack is generated in about 50% of the number of subjects even if the autoclave processing of the number of times at this level is performed.

By making the adhesive layers into the three-layer structure in this way, a stress is concentrated on the middle third adhesive material layer 97 even under severe conditions as in the autoclave processing. This can prevent generation of a crack in the respective layers and the respective interfaces other than the third adhesive material layer 97 and can further improve the durability of the sealing structure.

If the adhesive material layers are made into a structure of three or more layers as shown in Fig. 11, many interfaces between the materials are present between the tip rigid portion and the optical member compared to the two-layer structure. Therefore, it is usually believed that a stress is concentrated on these interfaces and a crack is easily generated.

However, the inventor has found out from the result shown in Fig. 12 that the adhesive layers are made into the three-layer structure, whereby the stress concentration to the interfaces is quite relieved and the stress is concentrated on the middle adhesive material layer.

Since a crack is easily generated at the interfaces, the stresses applied to the interfaces can be reduced. Accordingly, even when the adhesive layers are made into a structure of two or more layers so as to increase the interfaces, generation of a crack in the respective interfaces can be suppressed effectively, and durability and heat resistance can be further improved.

As mentioned above, the gap between the tip rigid portions 45 and the optical members 83 that is required for the medical electronic endoscopes is about 0.01 to 0.2 mm. By daring to provide the adhesive layers of the three-layer structure within such narrow thickness, it is possible to realize an unprecedented endoscope device having durability and heat resistance.

The present configuration is particularly useful for a connecting structure that has a large difference in thermal expansion coefficient between the tip rigid portion 45 and the optical member 83 and causes a larger distortion in the process of a temperature change.

Generally, when the thin sapphire plate 83A is fixed to the tip rigid portion 45 as shown in Fig. 8, it is difficult to make both with high dimensional accuracy and to accurately align both. However, by providing the third adhesive material layer 97 having viscosity between the first adhesive material layer 87 and the second adhesive material layer 89 as in the present configuration, the third adhesive material layer 97 absorbs this dimension error when combining the tip rigid portion 45 and the optical member 83. Hence, both can be easily combined with high accuracy.

Additionally, a thermal stress (heat distortion) to be generated can be further relieved by providing stretchability (ductility) to the third adhesive material. In order to provide the stretchability, for example, an aluminum alloy filler (made by Toyo Aluminum K.K.) made of aluminum metal powder for a filler with a particle size of 1 to 100 µm or desirably an alloy metal filler that is electrically insulated may be mixed in an adhesive material. Moreover, a black pigment, such as an isotropic graphite material may be mixed in order to prevent stray light.

The third adhesive material layer 97 may be further constituted by a plurality of layers having different thermal expansion coefficients. That is, the adhesive layers may be made into a structure of four or more layers. In this case, since stress can be concentrated on layers other than adhesive material layers at both ends, stress applied to the respective layers can be decentralized, and heat resistance and durability can be further improved as compared to the case of the three layers.

### <Third Configuration Example>

Next, a configuration in which a stepped portion is formed in the opening hole of the tip rigid portion and the optical member is fixed to this stepped portion will be described.

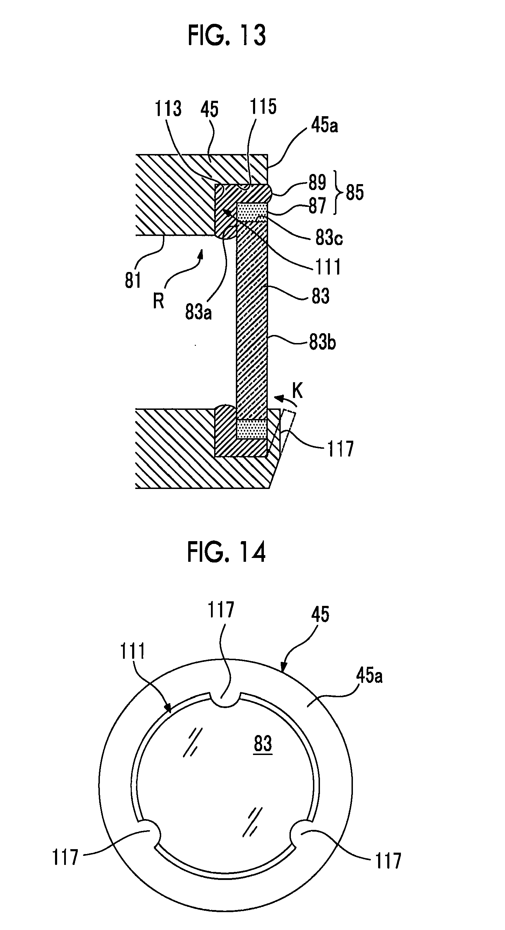

Fig. 13 is a schematic cross-sectional view showing that the optical member 83 is fixed to a stepped portion 111 of the tip rigid portion 45 via the plurality of adhesive layers 85.

In the present configuration, the stepped portion 111 that is enlarged in diameter by stepping is formed at one end portion of the inner peripheral surface of the opening hole 81 of the tip rigid portion 45. The stepped portion 111 is formed with an annular side surface 113 that faces the opening side of the opening hole 81, and a diameter-enlarged inner peripheral surface 115 at a diameter-enlarged inner peripheral portion.

Outer peripheral edges of the adhesive layers 85 and the optical member 83 are arranged in a region where the diameter of the opening hole 81 is enlarged by the stepped portion 111. The first adhesive material layer 87 is formed on the outer peripheral surface of the optical member 83 over the whole circumference thereof, and the second adhesive material layer 89 is formed on the annular side surface 113 and the diameter-enlarged inner peripheral surface 115 of the stepped portion 111 over the whole circumferences thereof.

The second adhesive material layer 89 is joined to the first adhesive material layer 87, and an outer peripheral edge 83a of the optical member 83 that faces the annular side surface 113. That is, the outer peripheral edge 83a of the optical member 83 that faces the annular side surface 113 is joined to the second adhesive material layer 89 over the whole circumference of the outer peripheral edge 83a, and forms a sealing structure between the tip rigid portion 45 and the optical member 83.

Additionally, an outer surface 83b of the optical member 83 is arranged so as to be flush with a tip surface 45a of the tip rigid portion 45, and the optical member 83 performs crimping to the tip rigid portion 45 with a latching claw 117 that protrudes in a plastically deformable manner and is held within the stepped portion 111. The latching claw 117 biases the optical member 83 in the plate thickness direction by pressurizing and deforming as shown by arrow K in the drawing. As shown in a front view of the optical member 83 in Fig. 14, at least three latching claws 117 are provided at the tip rigid portion 45 to prevent falling of the optical member 83. The latching claws 117 stably hold the optical member 83 within the stepped portion 111, and make it easy to fix the optical member 83 flatly to the tip surface 45a of the tip rigid portion 45.

The process of fixing the optical member 83 to the stepped portion 111 of the tip rigid portion 45 as described above is the same as the aforementioned process, and an example thereof will be shown below. (1) The paste-like first adhesive material is coated on the outer peripheral surface 83c of the optical member 83, and is temporarily fixed by heating at a temperature lower than the glass softening point. (2) The paste-like second adhesive material is coated on the opening hole of the tip rigid portion. (3) The optical member 83 formed with the first adhesive material layer 87 is fitted into the stepped portion 111 of the tip rigid portion 45. (4) The optical member 83 is supported by the latching claws. (5) Baking is performed at a temperature equal to or higher than the glass softening point.

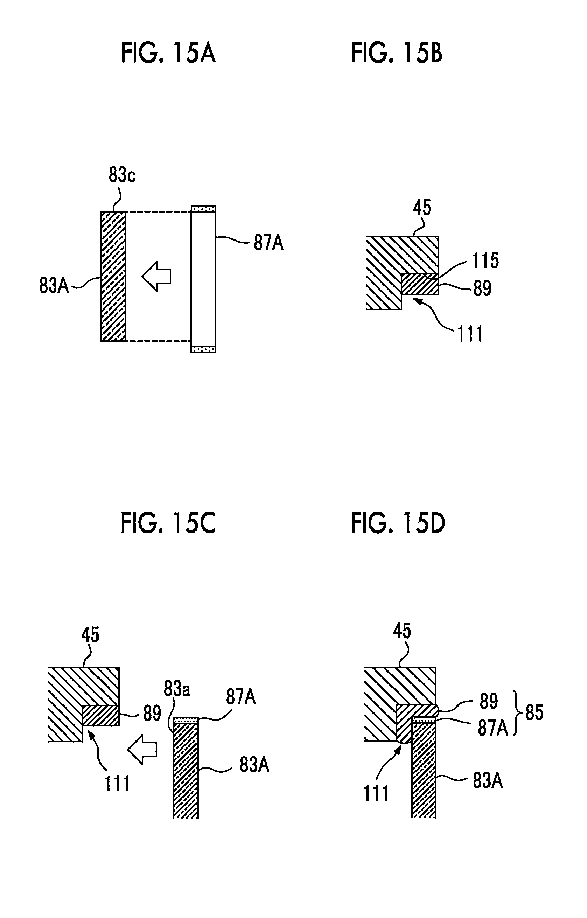

Additionally, when the sapphire plate is used as the optical member 83, a process shown in Figs. 15A to 15D can be adopted. (1) The first adhesive material preform 87A is attached to the outer peripheral surface 83c of the sapphire plate 83A, and is temporarily fixed by heating. (2) The paste-like second adhesive material is coated on the diameter-enlarged inner peripheral surface 115 of the tip rigid portion 45. (3) The sapphire plate 83A to which the preform 87A is temporarily fixed is fitted into the stepped portion 111 of the tip rigid portion 45. Accordingly, the second adhesive material spreads to the annular side surface 113 of the tip rigid portion 45, and the outer peripheral edge 83a of the sapphire plate 83A that faces the annular side surface 113. (4) The optical member 83 is supported by the latching claws. (5) Baking is performed at a temperature equal to or higher than the glass softening point.

According to the sealing structure between the tip rigid portion 45 and the optical member 83 (83A) that is formed by the above process, the optical member 83 (83A) is joined to the tip rigid portion 45 on two surfaces including the outer peripheral surface 83c thereof and the outer peripheral edge 83a. Thus, sealing performance and durability are improved.

In addition, when the adhesive layers are made into the three-layer structure, for example, the third adhesive material preform is attached to the outer peripheral surface of the first adhesive material preform 87A and temporarily fixed by heating after the preform 87A in Fig. 15A is formed. Then, the processes after Fig. 15B may be performed.

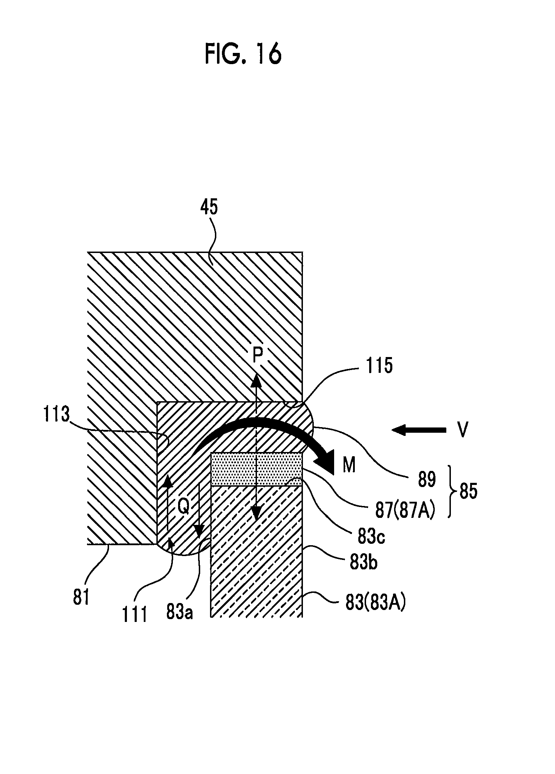

The effects of the above sealing structure will be described with reference to Fig. 16. Fig. 16 is an enlarged explanatory view showing an R portion of Fig. 13 in an enlarged manner. When temperature changes have occurred in the respective members, thermal stresses according to the thermal expansion coefficients of the respective members are generated. The thermal expansion coefficients have a relationship of the thermal expansion coefficient α_{OP} of the optical member 83 (83A) < the thermal expansion coefficient α₁ of the first adhesive material < the thermal expansion coefficient α₂ of the second adhesive material < the thermal expansion coefficient α_{f} of the tip rigid portion 45 as mentioned above. Thus, during a temperature rise, a tensile stress P is generated in the radial direction of the optical member 83 (83A) and a shearing stress Q is generated at the outer peripheral edge 83a of the optical member 83 (83A).

The shearing stress Q is generated over the whole circumference of the outer peripheral edge 83a of the optical member 83 (83A), and acts as a bending moment M that is going to warp the optical member 83 (83A) from the outside of the opening hole 81 toward the inside thereof. Heat distortion accompanying a temperature change is decentralized on the entirety of the adhesive layers 85 and the entire optical member 83 (83A) by the action of this bending moment M.

The total thermal stress energy at this time is the sum of the energy that generates the bending moment M due to the shearing stress Q and the energy of the tensile stress P. Accordingly, as compared to a case where only the outer periphery end portion of the optical member is joined as shown in Fig. 5, the tensile stress P decreases relatively by the amount of the total thermal stress energy that is decentralized to the shearing stress Q.

For this reason, the tensile stress P and the shearing stress Q are suppressed to a relatively low stress level by the decentralization of the total thermal stress energy, and even when there is a large amount of temperature change, breaking or damage does not occur easily.

Additionally, by adopting the above sealing structure, whether or not defects, such as a crack, have occurred in the adhesive layers 85 can be visually recognized through the outer peripheral edge of the optical member 83 (83A) when being observed in the direction of V shown in Fig. 16. This enables a joined state between both to be easily confirmed.

According to the endoscopes of the respective configuration examples described above, sealing performance, heat resistance, chemical resistance, and mechanical durability capable of responding to autoclave cleaning, are obtained simultaneously. In the autoclave cleaning, heating to about 160° C to 180°C is performed, pressurizing to about 2 bars is performed, and sterilizing and chemical cleaning are performed. When heat distortion increases with a temperature rise from the start of cleaning, and a preset temperature is reached, the amount of heat deformation from room temperature becomes the maximum. The thermal stress between the optical member 83 and the tip rigid portion 45 at this time become the maximum.

In the present configuration, the plurality of adhesive layers, such as the first adhesive material layer and the second adhesive material layer, are provided between the optical member 83 and the tip rigid portion 45, and the thermal expansion coefficients of the respective adhesive layers have a distribution that increases in a staircase pattern from the optical member 83 to the tip rigid portion 45 as an example. For this reason, even if the endoscope is exposed to a high-temperature environment as in the autoclave cleaning, the thermal stress to be generated is reduced, and rupture or damage of the respective adhesive layers or cracking or deformation of the optical member 83 or the tip rigid portion 45 can be prevented from occurring.

Similar to the thermal stress described above, not only a thermal stress caused by a change in environmental temperature of the endoscope but also a thermal stress that occurs during heating and a temperature rise of sintering processing of the tip rigid portion 45 and the optical member 83 or during a temperature drop can be reduced similarly.

The invention is not limited to the above embodiment, and combinations of the respective configurations of the embodiment or alternations or applications by those skilled in the art, which are based on the description of the specification and well-known techniques, are scheduled by the invention and included in the scope to be protected.

For example, the low-melting-point glass may be constituted by silicate glass in addition to bismuth-based glass. In order to control the thermal expansion coefficients or the like, additives, such as zinc oxide, alumina, diboron trioxide, and silica, can also be added to the low-melting-point glass. In addition, when phosphate glass or alkali-borate glass is used, water resistance can be improved by adding alkaline earths. The thermal expansion coefficients can be raised by adding magnesium oxide to an adhesive layer formed on a metal side with a higher thermal expansion coefficient. Additionally, the thermal expansion coefficients can be lowered by adding a small amount of tin oxide to an adhesive layer formed on the optical member side.

Although the endoscope has been illustrated and described above as a medical device, the invention is not limited to the medical endoscopes in which electronic components, such as the imaging element, are built in the tip portion, and may also be applied to various medical devices, such as a rigid scope, a scope endoscope, and various kinds of surgical equipment.

### [Example 1]

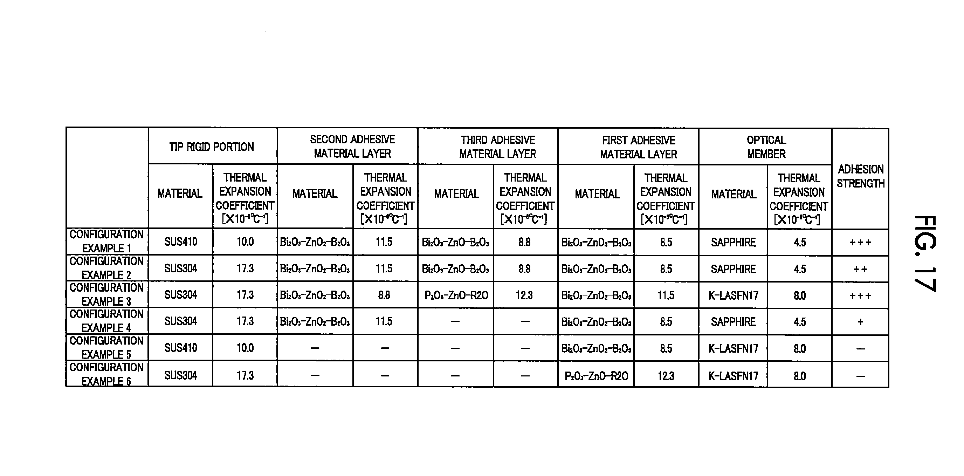

Fig. 17 is a view showing experiment examples of Configuration Examples 1 to 6 of the sealing structure between the optical member and the tip rigid portion. The configurations of Configuration Examples 1 to 6 and evaluation of adhesion strength thereof will be described below.

In Configuration Example 1, the tip rigid portion is stainless steel (SUS410) in which the thermal expansion coefficient α_{f} is 10.0 [× 10^{-6°}C⁻¹] and the optical member is a sapphire plate in which the thermal expansion coefficient α_{OP} is 4.5 [× 10⁻⁶°C⁻¹].

Additionally, the adhesive layers have a laminated structure of three layers. As the second adhesive material layer, an adhesive material of glass paste (thermal expansion coefficient α₁: 11.5 [× 10⁻⁶°C⁻¹], and baking temperature: 450°C) containing Bi₂O₃-ZnO₂-B₂O₃-based glass frit was formed at a temperature equal to or lower than the glass softening point. As the first adhesive material layer, an adhesive material of glass paste (thermal expansion coefficient α₂: 8.5 [×10⁻⁶°C⁻¹], and baking temperature: 450°C) containing Bi₂O₃-ZnO₂-B₂O₃-based glass frit was formed at a temperature equal to or lower than the glass softening point. The third adhesive material layer was formed by sintering an adhesive material of glass paste (thermal expansion coefficient α_{3:} 8.8 [× 10⁻⁶°C⁻¹], and baking temperature: 480°C) containing Bi₂O₃-ZnO₂-B₂O₃-based glass frit together with the first and second adhesive material layers at a temperature slightly exceeding the baking temperature.

Configuration Example 2 is the same as Configuration Example 1 except that the tip rigid portion is made of stainless steel (SUS304) in which the thermal expansion coefficient α_{f} is 17.3 [× 10⁻⁶°C⁻¹].

Configuration Example 3 is a configuration in which, in Configuration Example 1, the optical member is made of glass of L-LASFN (trade name: made by Sumita Optical Glass Inc.), the first adhesive material layer is formed from an adhesive material in which the thermal expansion coefficient α₂ is 11.5 [× 10⁻⁶°C⁻¹] and the baking temperature is 450°C, the second adhesive material layer is formed from an adhesive material in which the thermal expansion coefficient α₂ is 8.8 [× 10⁻⁶°C⁻¹] and the baking temperature is 450°C, and the third adhesive material is added between the first adhesive material layer and the second adhesive material layer. The third adhesive material layer is formed from glass paste containing P₂O₅-ZnO-R₂O-based glass frit, in which the thermal expansion coefficient α₂ is 12.3 [× 10⁻⁶°C⁻¹] and the baking temperature is 480°C.

Configuration Example 4 is a configuration in which, in Configuration Example 1, the tip rigid portion is made of stainless steel (SUS304) in which the thermal expansion coefficient α_{f} is 17.3 [× 10⁻⁶°C⁻¹] and the third adhesive material layer is omitted. The respective adhesive material layers that constitute the adhesive layers were heated and formed at a temperature equal to or lower than the glass softening point after the coating of the glass paste, and then, both were combined and sintered at a temperature slightly exceeding the baking temperature.

Configuration Example 5 is a configuration in which the tip rigid portion is made of stainless steel (SUS410), the optical member is made of glass of L-LASFN17 (trade name: made by Sumita Optical Glass Inc.), and the same first adhesive material layer as that of Configuration Example 1 is provided as a single-layer without providing the second adhesive material layer.

Configuration Example 6 is a configuration in which, in Configuration Example 5, the tip rigid portion is made of SUS304 and the first adhesive material layer is formed from glass paste containing P₂O₅-ZnO-R₂O-based glass frit, in which the thermal expansion coefficient α₂ is 12.3 [× 10⁻⁶°C⁻¹] and the baking temperature is 480°C.

The results obtained by evaluating the adhesion strength of a window (an observation window or an irradiation window) before and after execution of 500 times of the sterilization processing regarding the above Configuration Examples 1 to 6 are shown in Fig. 17.

In the evaluation of the adhesion strength, a case where the strength change from an initial state is < 5% (a change is not found) is represented by +++, a case where the strength change is < 10% is represented by ++, a case where the strength change is < 50% is represented by +, and a case where clouding or cracking is caused in a low-melting-glass portion is represented by -.

As can be understood from the comparison between Configuration Example 4 and Configuration Examples 5 and 6, durability and heat resistance can be improved by making the adhesive materials into the two-layer structure.

As can be understood from the comparison between Configuration Examples 1 to 3 and Configuration Example 4, durability and heat resistance can be further improved by making the adhesive materials into the three-layer structure.

As described above, the following matters are disclosed in the present specification.

The disclosed electronic endoscope device is an electronic endoscope device including: an optical member; and a frame body made of a material having a thermal expansion coefficient different from the optical member and holding the optical member. The optical member and the frame body are arranged at a tip portion of an insertion section inserted into a subject. An electronic component including an imaging element is built in the tip portion. The optical member and the frame body are connected to each other via three or more adhesive layers having mutually different thermal expansion coefficients.

In the disclosed electronic endoscope device, the three or more adhesive layers are made of low-melting-point glass, respectively.

In the disclosed electronic endoscope device, the low-melting-point glass is constituted by bismuth-based glass.

In the disclosed electronic endoscope device, the three or more adhesive layers are formed at least between an outer peripheral surface of the optical member formed in a plate shape and an inner peripheral surface of the frame body that faces the outer peripheral surface.

In the disclosed electronic endoscope device, one end portion of the inner peripheral surface of the frame body is formed with a stepped portion that is enlarged in diameter by stepping, and any one layer among the three or more adhesive layers is provided on an annular side surface formed by the stepping of the frame body over the whole circumference of the annular side surface, and an outer peripheral edge of the optical member that faces the annular side surface is joined to the one layer over the whole circumference of the outer peripheral edge.

In the disclosed electronic endoscope device, the frame body is made of an Fe-based material containing at least any one ofNi and Co.

In the disclosed electronic endoscope device, the optical member is made of sapphire.

The disclosed manufacturing method of an electronic endoscope device is a manufacturing method of an electronic endoscope device including an optical member and a frame body made of a material having a thermal expansion coefficient different from the optical member and holding the optical member, the optical member and the frame body being arranged at a tip portion of an insertion section inserted into a subject, and an electronic component including an imaging element being built in the tip portion. The method carries out at least, when the optical member and the frame body are connected to each other via three or more adhesive layers having mutually different thermal expansion coefficients, which are laminated in a thickness direction of a gap formed from an outer peripheral surface of the optical member and an inner peripheral surface of the frame body, a first adhesive material layer forming step of forming a first adhesive material layer on the outer peripheral surface side of the optical member; a second adhesive material layer forming step of forming a second adhesive material layer on the inner peripheral surface side of the frame body; a third adhesive material layer forming step of forming a third adhesive material layer on at least any one of the outer peripheral surface side of the first adhesive material layer and the inner peripheral surface side of the second adhesive material layer; an inserting step of inserting the optical member into the frame body after the first adhesive material layer forming step, the second adhesive material layer forming step, and the third adhesive material layer forming step; and a sintering step of sintering the frame body and the optical member via the adhesive material layers including the first adhesive material, the second adhesive material, and the third adhesive material after the inserting step.

In the disclosed manufacturing method of an electronic endoscope device, each of the adhesive materials contains low-melting-point glass frit as a filler.

In the disclosed manufacturing method of an electronic endoscope device, the first adhesive material layer forming step is a step of baking glass by heating to form the first adhesive material layer, after a paste-like first adhesive material is coated on the outer peripheral surface of the optical member.

In the disclosed manufacturing method of an electronic endoscope device, the first adhesive material layer forming step is a step of fitting an inner peripheral surface of a preform, which is obtained by annularly molding the first adhesive material, into the outer peripheral surface of the optical member, so as to form the first adhesive material layer.

In the disclosed manufacturing method of an electronic endoscope device, the second adhesive material layer forming step is a step of baking glass by heating to form the second adhesive material layer, after the paste-like second adhesive material is coated on the inner peripheral surface of the frame body.

In the disclosed manufacturing method of an electronic endoscope device, the second adhesive material layer forming step is a step of fitting an outer peripheral surface of a preform, which is obtained by annularly molding the second adhesive material, into the inner peripheral surface of the frame body, so as to form the second adhesive material layer.

In the disclosed manufacturing method of an electronic endoscope device, the second adhesive material layer forming step is a step of providing a stepped portion, which is enlarged in diameter by stepping, at one end portion of the inner peripheral surface of the frame body, and forming the second adhesive material layer on the diameter-enlarged inner peripheral surface of the stepped portion, the third adhesive material layer forming step is a step of forming the third adhesive material layer outside the first adhesive material layer. In the inserting step, the outer peripheral surface of the optical member is pressed against the second adhesive material layer, which is formed on the diameter-enlarged inner peripheral surface of the stepped portion, from an insertion direction of the optical member whereby the second adhesive material layer is made to extend to an annular side surface of the frame body formed by the stepping, and the second adhesive material layer is formed at a gap between the outer peripheral surface of the optical member and the diameter-enlarged inner peripheral surface of the stepped portion that faces the outer peripheral surface and a gap between the annular side surface and an outer peripheral edge of the optical member that faces the annular side surface over the whole circumferences thereof, respectively.

According to the electronic endoscope device of the invention, heat resistance and durability under severe conditions, such as autoclave processing, can be enhanced.

## Claims

1. An electronic endoscope device comprising:
an optical member; and
a frame body made of a material having a thermal expansion coefficient different from the optical member and holding the optical member,
the optical member and the frame body being arranged at a tip portion of an insertion section inserted into a subject,
wherein an electronic component including an imaging element is built in the tip portion, and
wherein the optical member and the frame body are connected to each other via three or more adhesive layers having mutually different thermal expansion coefficients.

2. The electronic endoscope device according to Claim 1,
wherein the three or more adhesive layers are made of low-melting-point glass, respectively.

3. The electronic endoscope device according to Claim 2,
wherein the low-melting-point glass is constituted by bismuth-based glass.

4. The electronic endoscope device according to any one of Claims 1 to 3,
wherein the three or more adhesive layers are formed at least between an outer peripheral surface of the optical member formed in a plate shape and an inner peripheral surface of the frame body that faces the outer peripheral surface.

5. The electronic endoscope device according to Claim 4,
wherein one end portion of the inner peripheral surface of the frame body is formed with a stepped portion that is enlarged in diameter by stepping, and
wherein any one layer among the three or more adhesive layers is provided on an annular side surface formed by the stepping of the frame body over the whole circumference of the annular side surface, and an outer peripheral edge of the optical member that faces the annular side surface is joined to the one layer over the whole circumference of the outer peripheral edge.

6. The electronic endoscope device according to any one of Claims 1 to 5,
wherein the frame body is made of an Fe-based material containing at least any one of Ni and Co.

7. The electronic endoscope device according to any one of Claims 1 to 6,
wherein the optical member is made of sapphire.

8. A manufacturing method of an electronic endoscope device including an optical member and a frame body made of a material having a thermal expansion coefficient different from the optical member and holding the optical member, the optical member and the frame body being arranged at a tip portion of an insertion section inserted into a subject, and an electronic component including an imaging element being built in the tip portion, the method carrying out at least,
when the optical member and the frame body are connected to each other via three or more adhesive layers having mutually different thermal expansion coefficients, which are laminated in a thickness direction of a gap formed from an outer peripheral surface of the optical member and an inner peripheral surface of the frame body,
a first adhesive material layer forming step of forming a first adhesive material layer on the outer peripheral surface side of the optical member;
a second adhesive material layer forming step of forming a second adhesive material layer on the inner peripheral surface side of the frame body;
a third adhesive material layer forming step of forming a third adhesive material layer on at least any one of the outer peripheral surface side of the first adhesive material layer and the inner peripheral surface side of the second adhesive material layer;
an inserting step of inserting the optical member into the frame body after the first adhesive material layer forming step, the second adhesive material layer forming step, and the third adhesive material layer forming step; and
a sintering step of sintering the frame body and the optical member via the adhesive material layers including the first adhesive material, the second adhesive material, and the third adhesive material after the inserting step.

9. The manufacturing method of an electronic endoscope device according to Claim 8,
wherein each of the adhesive materials contains low-melting-point glass frit as a filler.

10. The manufacturing method of an electronic endoscope device according to Claim 9,
wherein the first adhesive material layer forming step is a step of baking glass by heating to form the first adhesive material layer, after the paste-like first adhesive material is coated on the outer peripheral surface of the optical member.

11. The manufacturing method of an electronic endoscope device according to Claim 8 or 9,
wherein the first adhesive material layer forming step is a step of fitting an inner peripheral surface of a preform, which is obtained by annularly molding the first adhesive material, into the outer peripheral surface of the optical member, so as to form the first adhesive material layer.

12. The manufacturing method of an electronic endoscope device according to Claim 9,
wherein the second adhesive material layer forming step is a step of baking glass by heating to form the second adhesive material layer, after the paste-like second adhesive material is coated on the inner peripheral surface of the optical member.

13. The manufacturing method of an electronic endoscope device according to Claim 8 or 9,
wherein the second adhesive material layer forming step is a step of fitting an outer peripheral surface of a preform, which is obtained by annularly molding the second adhesive material, into the inner peripheral surface of the frame body, so as to form the second adhesive material layer.

14. The manufacturing method of an electronic endoscope device according to any one of Claims 8 to 13,
wherein the second adhesive material layer forming step is a step of providing a stepped portion, which is enlarged in diameter by stepping, at one end portion of the inner peripheral surface of the frame body, and forming the second adhesive material layer on the diameter-enlarged inner peripheral surface of the stepped portion,
wherein the third adhesive material layer forming step is a step of forming the third adhesive material layer outside the first adhesive material layer, and
wherein in the inserting step, the outer peripheral surface of the optical member is pressed against the second adhesive material layer, which is formed on the diameter-enlarged inner peripheral surface of the stepped portion, from an insertion direction of the optical member whereby the second adhesive material layer is made to extend to an annular side surface of the frame body formed by the stepping, and the second adhesive material layer is formed at a gap between the outer peripheral surface of the optical member and the diameter-enlarged inner peripheral surface of the stepped portion that faces the outer peripheral surface and a gap between the annular side surface and an outer peripheral edge of the optical member that faces the annular side surface over the whole circumferences thereof, respectively.
